# EUROPEAN PATENT APPLICATION

(11) **EP 3 403 643 A1**
(43) Date of publication of application: **21.11.2018**
(21) Application number: 17738073.0
(22) Date of filing: 06.01.2017
(51) Int. Cl.: A61K 9/00, A61K 9/70, A61K 38/28

(54) **PHARMACEUTICAL FORM FOR ORAL ADMINISTRATION OF A HIGHLY CONTROLLED AND STABLE DOSE OF NANOPARTICLES OR BIOMACROMOLECULE SUSPENSIONS**

(30) Priority: 15.01.2016 CL 2016112
(71) Applicant: Universidad De Chile, Santiago (CL)
(72) Inventor: MORALES MONTECINOS, Javier Octavio, Independencia Santiago (CL); MONTENEGRO NICOLINI, Miguel, Independencia Santiago (CL); CAMPANO HANTSCHERUK, Felipe Aaron, Independencia Santiago (CL)
(74) Representative: Isern Patentes y Marcas S.L.
(86) International application number: PCT/CL2017/050001
(87) International publication number: WO 2017/120689

(57) **Abstract**

The invention relates to the pharmaceutical industry, particularly to the pharmaceutical industry related to drugs comprising biomacromolecules, or biopharmaceuticals. Even more particularly, the invention relates to a pharmaceutical form comprising biomacromolecules such as lymphokines, hormones, haematopoietic factors, growth factors, antibodies, enzymes, inhibitors, vaccines, and DNA or RNA derivatives. The invention provides a pharmaceutical form comprising biomacromolecules and a method for producing same based on inkjet printing using inks formed by nanosystems comprising the biomacromolecule(s), the drug or biopharmaceutical being administered orally. Even more particularly, the invention relates to a pharmaceutical form for oral administration of a highly controlled, stable controlled release dose of a biomacromolecule comprising: a) a polymer film as a printing substrate, formed by at least one pharmaceutically acceptable excipient; and b) an inkjet ink printed on the polymer film and comprising nanoparticles or nanoparticle suspensions comprising the biomacromolecule and at least one pharmaceutically acceptable excipient.

## Description

### Field of the invention

The present invention relates to the pharmaceutical industry. In particular, with the pharmaceutical industry related to medicines comprising biologics. In particular, the present invention relates to a dosage form comprising biologics such as lymphokines, hormones, hematopoietic factors, growth factors, antibodies, enzymes, inhibitors, vaccines, DNA or RNA derivatives.

The present invention proposes a dosage form comprising biologics and a manufacturing method of said dosage form based on inkjet printing where said inks consist of nanosystems comprising the biologics, wherein said medicine or biologic is for buccal administration.

In particular, the present invention relates to a dosage form for buccal administration of highly controlled dosing, with a controlled and stable release of a biologic comprising: a. a polymer film as printing substrate consisting of at least one pharmaceutically acceptable excipient and b. an ink comprising nanoparticles or nanoparticle suspensions comprising said biologic and at least one pharmaceutically acceptable excipient printed on the polymer film.

### Background of the invention

Biologics such as insulin, antibodies, DNA or RNA derivatives, proteins or enzymes are administered only as injectables since they are very sensitive to the conditions of the gastrointestinal tract. Because of this, even if the oral route of administration would be preferred for biologics, such route is limited. Finding alternative routes to injectables has been a research focus for years without success. The present invention solves this pending task and proposes an alternative route to the injectable or oral route, for biologics.

Thus, the present invention proposes the buccal administration route, through polymeric films for the administration of biologics. Although polymeric films have been explored and described in the prior art as detailed later herein, the present invention allows to fabricate films with highly controlled doses and in nanosystems that allow control over the release, stability, and drug delivery.

In this invention a biologic can be selected from those active agents included in a recombinant biologic as the latter is defined in Table 1 from Ryu JK, Kim HS, Nam DH. Current status and perspectives of biopharmaceutical drugs. Biotechnol Bioproc E. 2012 Oct 24; 17(5): 900-11. Then, among these biologics the following can be named: lymphokines, hormones, hematopoietic factors, growth factors, antibodies, enzymes, inhibitors and vaccines.

The lymphokines as used herein, include, among others: aldesleukin cytokine, the antineoplastic protein denileukin difititox, the recombinant interleukin Oprelvekin, interferon α1, interferon α2a, interferon-a2b, interferon β1a, interferon β1b, interferon γ1b, and the tumoral necrosis factor human-α1a (TNFα-1a) tasonermin.

Hormones as used herein, include, among others: human insulin, insulin lispro, insulin aspart, insulin glulisine, insulin glargine, insulin detemir, glucagon, somatropin, somatrem, follitropin-α, follitropin-β, choriogonadotropin-α, lutropin-α, calcitonin, teritapide, preotact, thyrotropin-a, nesiritide.

Hematopoietic factors as used herein include, among others: filgrastim, lenogastrim, sargramostim, molgramostim, epoetin-α, epoetin-β, γ epoetin-γ, darbepoetin-α.

Growth factors as used herein include, among others: mecasermin, rinfabate mecarsemin, nepiderin, becaplermin, palifermin, dibotermin-α, epotermin-α. Antibodies as used herein, include, among others: Fab fragments such as arcitumomab, digoxin Fab, abciximab, certolizumab; murine antibodies such as muramonab-CD3, capromab, ibritumomab tiuxetan, toistumomab; chimeric antibodies such as rituximab, infliximab, basiliximab, cetuximab, vedotin brentuximab; humanized antibodies such as daclizuman, trastuzumab, palivizumab, gemtuzumab ozogamicin, alemtuzumab, efalizumab, omalizumab, bevacizumab, natalizumab, ranbizumab, eculizumab, tocilizumb; human antibodies such as adalimumab, panitumimab, golimumab, canakinumab, ustekinumab, ofatumumab, denosumab, belimumab, ipilimumab.

Enzymes as used herein, include, among others: imiglucerase, agalsidase-β, alglucosidase-α, laronidase, aldusufase, galsulfase factor VIIa, factor VIII, factor IX, drotrecogin-a, alteplase, reteplase, teneceplase, dornase-a, rasburicase.
Inhibitors as used herein include, among others: desirudin, lepidurine, antithrombin III, ecallantide, anakinra.

Vaccines as used herein, include, among others: human hepatitis vaccine, human papilloma virus vaccine.
In particular, the biologic may be selected from lysozyme, ribonuclease, angiotensin 1-9 and insulin.

In addition, the polymer film comprises film forming polymers such as polyvinyl pyrrolidone, polyvinyl alcohol, chitosan, alginate, agar, carrageenan, guar gum, xanthan gum, polycarbophil, polyacrylic acid derivatives, and polymethacrylic acid derivatives.

Alternatives solutions to the proposed problem by the present invention and that use all other alternative administration routes (nasal, pulmonary, ocular, vaginal, rectal) do not use mucoadhesive polymer films. The skin, which could use films, does not allow passage of these molecules except for physical skin modifications (for example, generating pores). Research in biologics through the buccal route is focused on films obtained by methods quite different to that of the present invention or through sprays containing nanodroplets for absorption. For example, polymeric films consisting of a solid solution or dispersion of chitosan and ethylenediaminetetraacetic acid containing insulin have been described. This system does not contemplate the incorporation of insulin in nanoparticles, see Cui F, He C, He M, Tang C, Yin L, Qian F, et al. Preparation and evaluation of chitosan-ethylenediaminetetraacetic acid hydrogel films for the mucoadhesive transbuccal delivery of insulin. J Biomed Mater Res A. 2009; 89A (4): 1063-1071. In another example, the inventor in previous research (Morales JO, Ross AC, JT McConville Protein-coated nanoparticles embedded in films as delivery platforms. Journal of Pharmacy and Pharmacology 2013; 65 (6): 827-38; JO Morales, Huang S, Williams RO III, JT McConville Films loaded with insulin-coated nanoparticles (ICNP) as potential platforms for peptide buccal delivery Colloids and Surfaces B: Biointerfaces 2014 Oct 1; 122: 38-45; JT McConville, Morales JO, Ross AC Bioadhesive films for local and/or systemic delivery [Internet] WO2014065870 A1, 2014 [cited October 6 2014] Available from:... https://patents.google.com/patent/ WO2014065870A1) and in Giovino C , Ayensu I, J Tetteh, Boateng JS. Development and characterization of chitosan films impregnated with insulin loaded PEG-b-PLA nanoparticles (NPs): A potential approach for buccal delivery of macromolecules. Int J Pharm. 2012; 428 (1-2): 143-51) have described polymeric films incorporating nanoparticles loaded with peptides and proteins during the manufacturing process and casting. This order of procedure, it has been found that the biologic is exposed to contact with solvents, other excipients, and the film drying process. On the other hand, the incorporation of dispersed polymeric nanoparticles within chitosan polymer matrices for controlled insulin release has been described. These nanoparticles are incorporated to the system during the manufacturing process and the biologic is exposed to the process conditions (solvents and drying, for example). Finally, an insulin spray has recently been developed for buccal administration of the peptide (see Modi P, Mihic M, Lewin A. The evolving role of oral insulin in the treatment of diabetes using a novel RapidMist System. Diabetes/Metabolism Research and Reviews 2002; 18 (S1). S38-42; Guevara-Aguirre J, Guevara M, Saavedra J, Mihic M, Modi P. Beneficial effects of addition of oral insulin spray (Oralin) on insulin secretion and metabolic control in subjects with type 2 diabetes mellitus suboptimally controlled on oral hypoglycemic agents. Diabetes Technol Ther 2004 Feb; 6 (1):. 1 -8; Sadrzadeh N, Michael J. Glembourtt, Cynthia L. Stevenson Peptide drug delivery strategies for the treatment of diabetes. Journal of Pharmaceutical Sciences 2007; 96 (8): 1925-1954) which is also very different. Biologics printing as a technology has been described for other purposes (sensors) and in complete absence of nanosystems, which in the present invention is essential. For example, printing technology has been used to deposit DNA solutions and evaluate the effect of temperature on stability, see Suzuki, Nobuko and Yamamoto. 2000. "Microarray Fabrication with Covalent Attachment of DNA Using Bubble Jet Technology." Nature Biotechnology 18(4): 438-41. Other research has focused on demonstrating that the printing of bare biologics maintains its structure at least partially, see Zheng Q, Lu J, Chen H, Huang L, Cai J, Xu Z. Application of inkjet printing technique for biological material delivery and antimicrobial assays. Anal Biochem. 2011 Mar; 410 (2): 171-6. All these investigations have omitted the use of nanosuspensions as strategies to formulate biologics.

Biologics such as insulin, monoclonal antibodies or DNA and RNA derivatives, are almost entirely formulated and administered for injectable routes (intravenous, subcutaneous, or intramuscular) due to their limitations over other routes. The injectable route has a number of limitations, the most known being associated with pain management. Although easily approached, pain exercised when administering injectables can lead to late therapy start. For example, in diabetes treatment this phenomenon is known as psychological insulin resistance. More serious problems come hand in hand with the pharmacokinetics of injecting a biologic. These molecules, though diverse, may have reduced circulating half-life, which limits their use. Furthermore, injectables result in indiscriminate distribution, and can result in undesired side effects.

Historically, drugs are administered by the oral route. However, in all these, biologics that would comprise the medicine cannot be compressed into a tablet for patients to swallow due to a large number of constraints. The GI tract drug presents the drug with acidic pH conditions in the stomach, proteolytic enzymes along the tract, the presence of bile salts, and microbiota in the large intestine. These molecules are then prone to be degraded when administered by the oral route.

With all the limitations of the injectable and oral routes, pharmaceutical development in the administration of biologics focuses on finding alternative routes. Only two insulins administered by alternative routes have been approved by the FDA, the now retired Exubera (GS Mack. Pfizer dumps Exubera. Nat Biotech. 25 (12), 1331-1332 (2007)) and the currently marketed Afrezza (www.fda.gov/NewsEvents/Newsroom/PressAnnouncements/ucm403122.htm; www.afrezza.com). Both technologies have been developed for administration by the pulmonary route (with completely different dosage forms in relation to this invention). Outside these technologies, all development in biologics is focused on injectable formulations and research for alternative routes (there are now programs in Phase III).

One of the alternative routes of administration is the buccal route. The buccal administration of insulin, lysozyme, albumin has been studied but with the technology of McConville JT, Morales JO, Ross AC. WO2014065870A1 which lies significantly far from the present invention.

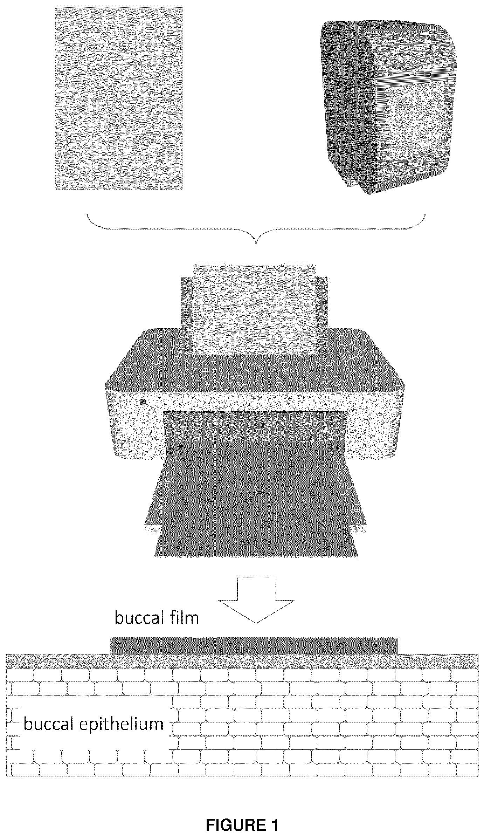

The present invention is a method of manufacturing a dosage form based on printing inks consisting of nanosystems containing as active ingredient a biologic selected from lymphokines, hormones, hematopoietic factors, growth factors, antibodies, enzymes, inhibitors, vaccines and DNA or RNA derivatives. In particular, the biologic may be selected from lysozyme, ribonuclease, angiotensin 1-9 and insulin. Once the printing process occurs, the dosage form is produced (polymer film loaded with the biologic) for subsequent buccal administration (Figure 1).

This printing method is highly predictable and reproducible for naked the biomacromolecule, and therefore, it is reproducible for nanosystems in inks.

Thus, the present invention uses nanosystems to generate the pharmaceutical product containing biologics for buccal use. In this way, limitations that do not have independent strategies are solved, because they have not been adequately addressed in the state-of-the-art.
Overall, biologics printing, is very poorly described in the literature. One of the newest articles describing printing of biologics is a review by lhalainen et al (lhalainen P, et al. Printing technologies for biomolecule and cell-based applications. International Journal of Pharmaceutics. In Press (2015)). In its section "3. Piezeoelectric and thermal inkjet technologies in biomolecule-based applications" the authors describe the thermal printing strategy but do not explore or suggest producing medicines, or nanosystems use to encapsulate biologics. In the present invention, the use of nanosystems is essential to form the dosage form (this provides a system with controlled release) and protect the biologic, which is generally described as one of the major constraints: heat and other ink ingredients exposure. Thus, only through nanosystems it is possible to separate the biologic of events that could provide destabilization along with the entire system of strict control over the release and delivery.

Other patent documents that may be mentioned in relation to the present invention are, for example, WO2014144512 (Apecia Pharmaceuticals Company) that teaches a form of 3D printed medicine of rapid dispersion and high doses of levetiracetam (anticonvulsant) which is in a porous matrix and that disperses in water in less than about 10 seconds. The document also discloses a process for preparing the dosage form. It does not relate to the manufacture of medicines based on inkjet printing of inks consisting in nanosystems containing the drug.

WO2014188079 (Abo Akademi University) relates to oral dosage forms of vitamin(s) and/or dietary mineral(s) or nicotine produced by printing techniques. It also relates to a method of producing an oral dose(s) of vitamin(s) and/or dietary mineral(s) or nicotine by printing techniques. It does not relate to the manufacture of medicines based on inkjet of printing inks consisting in nanosystems containing the drug.

TWI364442 (Du Pont) teaches a method for depositing a printable inkjet composition to a substrate comprising: depositing an ink composition on a substrate by inkjet printing; wherein said composition comprises: (a) functional material; (B) organic polymer comprising polyvinyl pyrrolidone; dispersed in (c) dispersion vehicle selected from organic solvent, water or mixtures thereof; and wherein the viscosity of said composition is between 5 mPa.s - 50 mPa.s at a temperature of 25 to 35°C. Moreover, TWI363081 teaches inkjet printing at least one patterned layer of the above composition in a substrate for fabricating an electronic component.

Then it was pending - in the prior art, the search for pharmaceutical forms for biologics for alternative routes to injectable and oral routes.

### Brief Description of the Invention

In particular, the present invention relates to a highly controlled dosage form for buccal dosing, with a controlled and stable release of a biologic comprising: a. a polymer film as printing substrate consisting of at least one pharmaceutically acceptable excipient and b. an ink printed on the polymeric film comprising nanoparticles or nanoparticle suspensions comprising said biologic and at least one pharmaceutically acceptable excipient,
wherein said biologic can be selected from lymphokines, hormones, hematopoietic factors, growth factors, antibodies, enzymes, inhibitors and vaccines.
wherein said lymphokine can be selected : aldesleukin cytokine, the antineoplastic protein denileukin difititox, the recombinant interleukin Oprelvekin, interferon α1, interferon α2a, interferon-α2b, interferon β1a, interferon β1b, interferon γ1b, and the tumoral necrosis factor human-α1a (TNFα-1a) tasonermin.
wherein said hormones may be selected from human insulin, insulin lispro, insulin aspart, insulin glulisine, insulin glargine, insulin detemir, glucagon, somatropin, somatrem, follitropin-α, follitropin-β, choriogonadotropin-α, lutropin-α, calcitonin, teritapide, preotact, thyrotropin-α, nesiritide.
wherein said hematopoietic factors may be selected filgrastim, lenogastrim, sargramostim, molgramostim, epoetin-α, epoetin-β, γ epoetin-γ, darbepoetin-α. wherein said growth factor can be selected mecasermin, rinfabate mecarsemin, nepiderin, becaplermin, palifermin, dibotermin-α, epotermin-α.
such antibodies may be selected from Fab fragments such as arcitumomab, digoxin Fab, abciximab, certolizumab; murine antibodies such as muramonab-CD3, capromab, ibritumomab tiuxetan, toistumomab; chimeric antibodies such as rituximab, infliximab, basiliximab, cetuximab, vedotin brentuximab; humanized antibodies such as daclizuman, trastuzumab, palivizumab, gemtuzumab ozogamicin, alemtuzumab,
efalizumab, omalizumab, bevacizumab, natalizumab, ranbizumab, eculizumab, tocilizumb; human antibodies such as adalimumab, panitumimab, golimumab, canakinumab, ustekinumab, ofatumumab, denosumab, belimumab, ipilimumab.
such enzymes can be selected from imiglucerase, agalsidase-β, alglucosidase-α, laronidase, aldusufase, galsulfase factor VIIa, factor VIII, factor IX, drotrecogin-α, alteplase, reteplase, teneceplase, dornase-α, rasburicase.
Such inhibitors can be selected desirudin, lepidurine, antithrombin III, ecallantide, anakinra.
such vaccines may be selected from human hepatitis vaccine, human papilloma virus vaccine. In particular, said biologic may be selected from lysozyme, ribonuclease, angiotensin 1-9 and insulin.
Furthermore, said polymeric film comprises film forming polymers such as polyvinyl pyrrolidone, polyvinyl alcohol, chitosan, alginate, agar, carrageenan, guar gum, xanthan gum, polycarbophil and polyacrylic acid derivatives.

Thus, the present invention relates to a dosage form that allows buccal administration of biologics. In general, biologics have limited capacity of absorption through the buccal mucosa and multiple strategies have been explored to allow administration. Among the various strategies developed to date for the above purpose polymer films may be mentioned as one of the most suitable buccal drug delivery system. The present invention discloses the manufacture of polymeric films as buccal delivery systems. This system is based on the development of nanosuspensions as biologics containing to be used as inks in an inkjet printing process.

First, the nanocarriers constituting the printing inks containing biologics are developed. To this end, nanoparticle fabrication and characterization was conducted by the following techniques: manufacturing of polymeric nanoparticles (also referred to as nanocomplexes or polyelectrolyte complexes), protein coated nanoparticles, nanoemulsions and nanocapsules.

For the development of polymeric nanoparticles, complex coacervation technique was used and it follows the electrostatic interaction of a polycation (a polymer with cationic monomer units) with a polyanion (a polymer with anionic monomeric units). Moreover, along a study of charge ratio (negative charges/positive charges, n⁻/n⁺), the formation of nanoparticles was observed only in presence of an excess of polyanion. The characterization of these systems is performed by dynamic light scattering (DLS) to study size distribution, and laser micro-Doppler electrophoresis (mLDE) for determining the zeta potential.

The influence of an absorption enhancer, and printing of nanosuspensions on polymer films as printing substrate were also determined.

Printing experiments on inert films of solutions of biologics allowed demonstrating the capacity of the printing system to deliver proportional amounts to the printed areas and concentrations thereof. Additionally, experimental studies of activity showed that the printing process can variably reduce the integrity of the drug and it was proportional to the amount printed. Similarly, the incorporation of an absorption enhancer had a negative effect on the integrity of the biologic, reducing its activity.

The experimental results of development of nanoemulsions and nanocapsules, searched to determine formulations useful as printing inks in terms of size, uniformity, encapsulation of Lys, and post-printing activity. The experimental design was conducted as a scan through a ternary diagram of how the composition results in different nanoemulsions conditions.

For purposes of inkjet printing, nanoemulsions properties allow that they are suitable for printing. Printing nanoemulsions loaded with lysozyme is possible and the physicochemical characteristics of the particles are kept upon completion of the printing process.

The printing process does not alter the physicochemical characteristics of polymeric nanoparticles obtained by coacervation of different charge ratios and different total charges on the number of positive and negative charges which provide each polymer forming the nanoparticle. A controlled and reduced release can always be maintained in the first 72 hours, regardless of the conditions, after subjecting the particles to the printing process. This profile ensures that such nanocarriers can carry the drug protected from environmental conditions (and the printing process) to the therapeutic target where it can be released. Polymeric nanoparticles show a typical spherical appearance.

After the printing process, the mechanical and morphological properties of the polymer films do not vary. The mucoadhesive properties are diminished, but do not prevent the use of the film as dosage form.

Experimental studies of polycation and polyanion (n⁻/n⁺) and the sum of total charges provided to the reaction to determine their influence on the characteristics of the nanoparticles show a tendency to lower average sizes as the charge ratio grows, relatively independent of the total charge amount. Smaller sizes become apparent from a charge ratio of 0.5, which coincides with that observed in the zeta potential, where the ratio of 0.5 is where charge inversion occurs from negative to positive.

The results of the polydispersity index indicate that the region between the charge ratio of 0.25 to 0.75 is the one combining smaller sizes, stronger zeta potential, and low polydispersity indices. Polymeric nanoparticles exhibit spherical morphology by scanning electron microscopy (SEM) and their size determined by dynamic light scattering (DLS) correlates to that of SEM.

The use of nanoparticles incorporated in conventional strategies on polymer films, such as buccal delivery system shows that the manufacture of protein coated nanoparticles can be developed by varying the molecule constituting the coprecipitation core.
In the process of coprecipitation only some materials that make up the core, such as the aforementioned saccharides, i.e, lactose, mannitol and sorbitol, proved useful.
As mentioned above for nanoemulslones and nanocapsules as lysozyme vehicles, the systems were obtained by the method of solvent displacement, where an organic phase containing oil (as an oil core of the nanostructure), a surfactant (such as lecithin, cetylpyridinium chloride, hexadecyltrimethylammonium bromide), and miscible organic solvents with water (such as acetone or ethanol), is incorporated into an aqueous solution to form a nanoemulslón (without agitation or high-energy homogenization). Particularly, lysozyme as a biologic drug example was used in nanoemulslones and nanocapsules as potential buccal delivery system. Then, printing inks containing lysozyme or ribonuclease (RNAse) as biologics showed that the process of printing allows to obtain controlled and linearly increasing doses with respect to the printed surface. Additionally, incorporating an absorption enhancing agent, such as deoxycholate, maintains the same linear trends in the process of printing lysozyme but with a decrease in the amount printed and its relative activity under the same conditions of surface and lysozyme concentration.
In general, such absorption enhancing agent can be selected from bile salts and derivatives, including deoxycholic acid, taurocholic acid, glycodeoxycholic acid, glycocholic acid and taurodeoxycholic acid.

Since the inks require the incorporation glycerol as a viscosity agent, the evaluation of lysozyme inks with or without glycerol was performed, and lysozyme does not change during the process.
Propylene glycol can also be used as viscosity agent.
The process of printing nanoparticles as carriers of biologic drug models was performed using polymeric nanoparticles and incorporated into the cartridge as a nanosuspension. The particle properties were measured before and after the printing process. Except an increase in the polydispersity index, the particles containing lysozyme as a model maintain their properties during the printing process and define the methodology for incorporating drug loaded nanoparticles on polymer films. Similar results were obtained for insulin-loaded polymeric nanoparticles in which a slight increase in particle size was observed, but always maintaining suitable polydispersity and size characteristics in the nanometer range.
The nanocarriers constituting the printing inks containing biologics are prepared by various techniques, including those selected from the group consisting of: manufacturing polymeric nanoparticles
(also referred to as nanocomplexes), protein coated nanoparticles
(protein-stabilized nanoparticles), nanoemulsions, and nanocapsules.

In developing polymeric nanoparticles, complex coacervation technique was used and it follows the electrostatic interaction of a polycation (a polymer with cationic monomer units) with a polyanion (a polymer with anionic monomeric units).

### Brief Description of the Figures

- **Figure 1.**: Diagram showing the disclosed process in general, for the manufacture of polymeric films loaded with biologics. Films are made as printing substrates and ink are formulated as a nanosuspension to allow proper dosing of the biologic. The printing process itself results in the medicine itself.
- **Figures 2A-2C.**: Evolution of particle size and polydispersity of polymeric nanoparticles loaded with lysozyme before/after printing on the inert surface.
- **Figures 3A and 3B.**: Printed samples with different levels of concentration (0.15, 0.5, 3.5, and 10 mg/mL) and various printing surfaces (4, 9, 16, and 49 cm²) for both pure lysozyme (A) and as a mixture with sodium deoxycholate (B).
- **Figures 4A-4C.**: Results of relative activity to Lys (a) and RNAse (b) with and without the addition of absorption enhancing agent deoxycholate (DCH) at different concentrations and printing surfaces (4, 9, 16, and 49 cm²).
- **Figure 5**.: SEM micrograph example of a nanoemulsion loaded with Lys. The bar represents 500 nm.
- **Figure 6A and 6B.**: Show the efficiency of association of nanoemulsions (NE) after being subjected to the printing process, and the effect of the printing process on the physicochemical characteristics of a subsequent NE print in different printing surface areas.

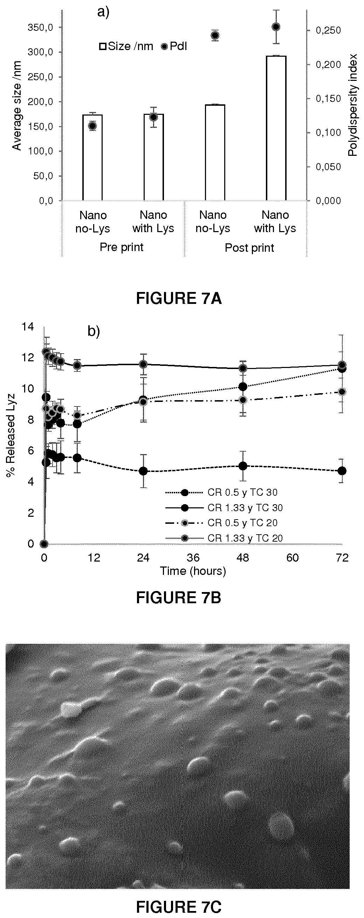

- **Figure 7A-7C.**: Show the effect of printing nanosuspensions as printing inks for Lys. The release profiles from different Lys polymeric nanoparticles formulations and an exemplification of how they are observed (by SEM, bar represents 500 nm).
- **Figure 8A-8C.**: (a and b) Effect of printing on nanosuspensions as insulin printing inks with a charge ratio of 0.5 (negative) and 2 (positive). (C) Efficiency of association of insulin in the printed formulations charge ratio 0.5 (negative) and 2 (positive).
- **Figure 9A-9C.**: Effect of the printing process on the mechanical (left) and mucoadhesive properties (right) of polymeric films of different grades of HPMC.
- **Figure 10A-10E.**: Study of the effect of charge ratio (n⁻/n⁺) and the total sum of loads (n⁻ + n⁺) on the average size (Figure 10a), zeta potential (Figure 10b) and polydispersity index (Figure 10c). SEM images of formulations are also observed for the n⁻ + n⁺= 6 µM series (Figure 10d and Figure 10e).
- **Figure 11A and 11B.**: Average size (Z-Average) and zeta potential of different formulations of nanoparticles precursors obtained by antisolvent co-precipitation. The SEM image shows an example of nanoparticles of D,L-valine typically obtained by this process (scale 500nm).
- **Figure 12A and 12B.**: Study of the linearity of the RNAse amount deposited depending on the printed surface and with or without the presence of deoxycholate.
- **Figure 13.**: FTIR spectra of lysozyme with glycerol (above) and lysozyme only (below) as model ink of the printing process.

### Detailed description of the invention

The present invention allows for a dosage form comprising biologics for an alternative route to oral and injectables. The present invention proposes the buccal administration route, through polymeric films, which allow making films with highly controlled doses and nanosystems which allows control over the release, stability, and delivery of the biologic, wherein said biologic can be selected from lymphokines, hormones, hematopoietic factors, growth factors, antibodies, enzymes, inhibitors and vaccines, wherein said lymphokine can be selected from aldesleukin cytokine, the antineoplastic protein denileukin difititox, the recombinant interleukin Oprelvekin, interferon α1, interferon α2a, interferon-α2b, interferon β1a, interferon β1b, interferon γ1b, and the tumoral necrosis factor human-α1a (TNFα-1a) tasonermin.
wherein said hormones may be selected from human insulin, insulin lispro, insulin aspart, insulin glulisine, insulin glargine, insulin detemir, glucagon, somatropin, somatrem, follitropin-α, follitropin-β, choriogonadotropin-α, lutropin-α, calcitonin, teritapide, preotact, thyrotropin-α, nesiritide.
wherein said hematopoietic factors may be selected filgrastim, lenogastrim, sargramostim, molgramostim, epoetin-α, epoetin-β, γ epoetin-γ, darbepoetin-α. wherein said growth factor can be selected mecasermin, rinfabate mecarsemin, nepiderin, becaplermin, palifermin, dibotermin-α, epotermin-α.
such antibodies may be selected from Fab fragments such as arcitumomab, digoxin Fab, abciximab, certolizumab; murine antibodies such as muramonab-CD3, capromab, ibritumomab tiuxetan, toistumomab; chimeric antibodies such as rituximab, infliximab, basiliximab, cetuximab, vedotin brentuximab; humanized antibodies such as daclizuman, trastuzumab, palivizumab, gemtuzumab ozogamicin, alemtuzumab, efalizumab, omalizumab, bevacizumab, natalizumab, ranbizumab, eculizumab, tocilizumb; human antibodies such as adalimumab, panitumimab, golimumab, canakinumab, ustekinumab, ofatumumab, denosumab, belimumab, ipilimumab.
such enzymes can be selected from imiglucerase, agalsidase-β, alglucosidase-α, laronidase, aldusufase, galsulfase factor VIIa, factor VIII, factor IX, drotrecogin-α, alteplase, reteplase, teneceplase, dornase-α, rasburicase.
Such inhibitors can be selected desirudin, lepidurine, antithrombin III, ecallantide, anakinra.
such vaccines may be selected from human hepatitis vaccine, human papilloma virus vaccine.
In particular, said biologic may be selected from lysozyme, ribonuclease, angiotensin 1-9 and insulin.
As an example, two biologics and two nanosystems were tested. Film printing films was assessed with different materials.
The present invention is a method of manufacturing a pharmaceutical dosage form based on printing inks consisting in nanosystems containing biologics. Once the printing process occurs, the medicine (polymer film incorporated with biologics) for subsequent buccal administration (Figure 1) is generated.

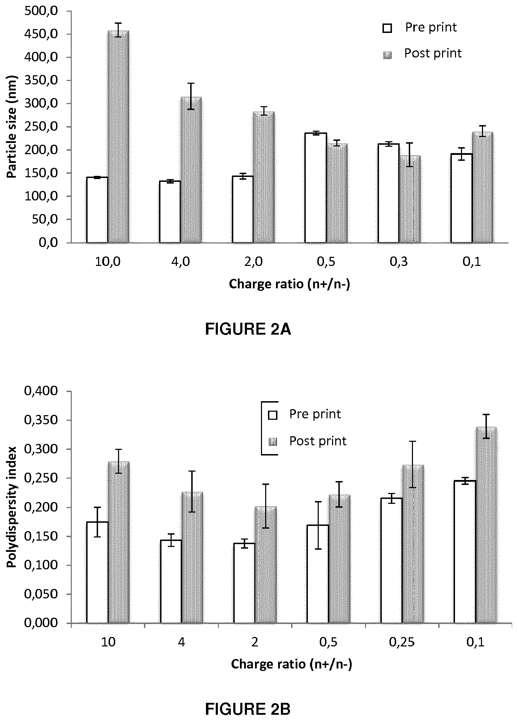

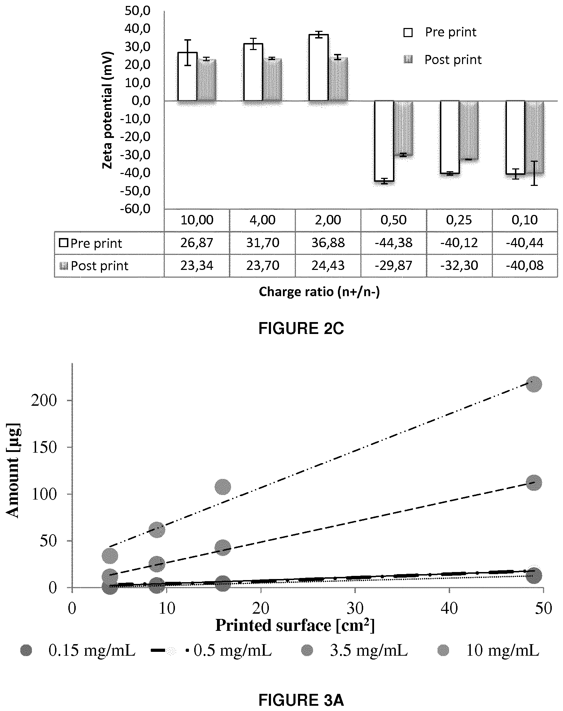

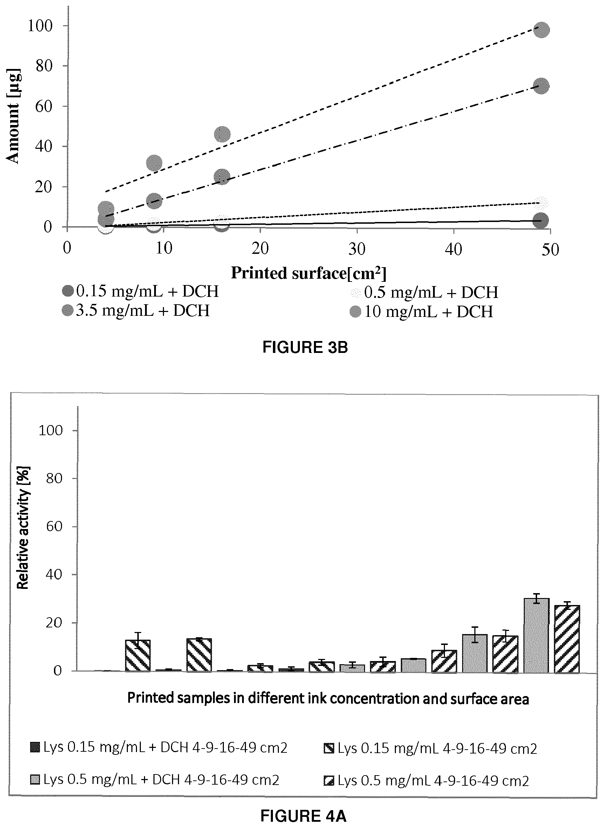

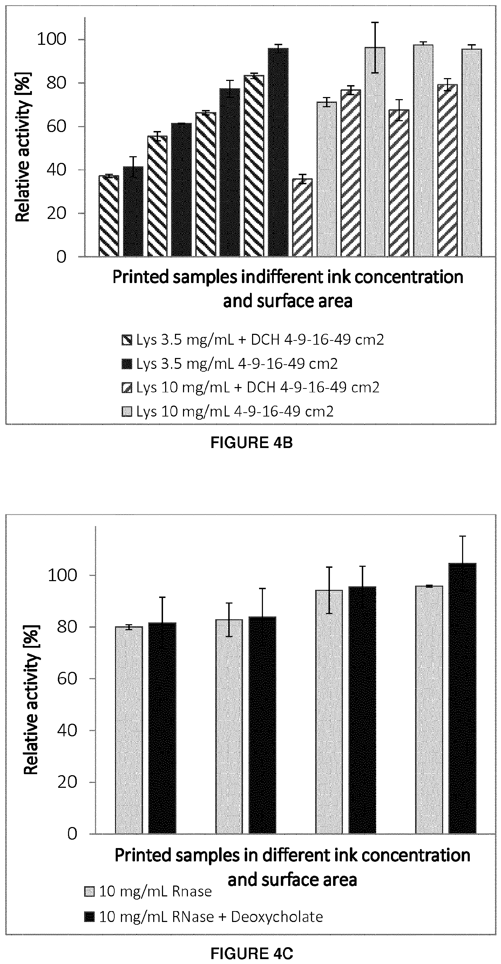

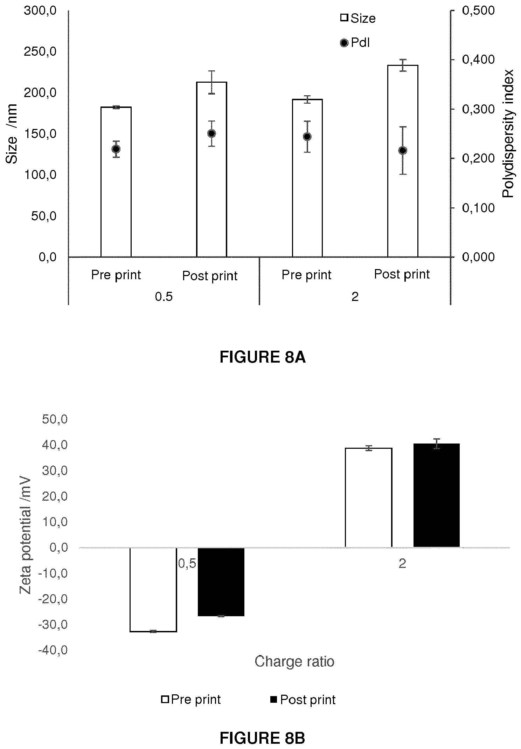

Results are available for the manufacturing process of the inks as well as polymeric structures (polymeric nanoparticles by coacervation) or emulsions (nanoemulsions of miglyol core). These inks have a particle size range between 100 and 300 nm depending on its nature, and a wide range of encapsulation efficiencies (50 to 95% of association). Studies show that release kinetics control the release of lysozyme (one biologic model) again dependent on the nature of the particle. One of the most remarkable and illustrating operation of the present invention data is shown in Figure 2. This corresponds to the example of a formulation before and after being subjected to the printing process and shows that the particle characteristics change little during the process. Load and protein activity studies of a biologic model (lysozyme) indicate that the amount immobilized at the particle together with the structure of the biologic are preserved during the printing process. Another notable example is seen in Figures 8A and 8B detailing the variation in size and polydispersity of two formulations of polymeric nanoparticles containing insulin. It is noted that the size increases in about 50 nm after the printing process, but always resulting in formulations with nanoparticles averaging sizes less than 250 nm.
This printing process is highly predictable and reproducible for naked biologics (e.g. lysozyme solutions, Figure 3) and therefore it is reproducible for nanosystems inks. Thus, the present invention uses nanosystems to generate the medicine comprising biologics for buccal use.
The present invention has its most obvious application in the pharmaceutical industry. All industries related to biologics could be beneficiaries of the present invention. Also, within the pharmaceutical industry, those industries using films as dosage forms (3M, www.3m.com/healthcare; LTS Lohmann, www.ltslohmann.de/en/home.html; BDSI, www.bdsi.com; among others), could also benefit from the present invention.
Early developments in this technology were focused on studying a range of types of nanoparticles: polymeric nanoparticles, nanoemulsions and nanocapsules, and protein coated nanoparticles. Due to the morphology and size achieved with the protein coated nanoparticles obtained by antisolvent coprecipitation (Le AD, Padilla A, Morales JO, McConville JT. Investigation of Antisolvent Manufacturing Co-precipitation of Protein-Coated Particle (PCP) Precursors using a Variable Injection Speed Linear Actuator in: 2015 AAPS Annual Meeting and Exposition Orlando, Florida, USA, 2015, and Le AD, Padilla a, Morales JO, McConville JT Formulation and Optimization of Protein-Coated Nanoparticles in: 42nd Annual Meeting and Exposition of the Controlled Release Society Edinburgh, Scotland; 2015), it was chosen to only continue with nanostructured inks based on polymeric nanoparticles through coacervation, nanoprecipitation, nanoemulsions and nanocapsules. The influence of an absorption enhancer, and printing on polymer films nanosuspensions as printing substrate was also determined. Printing experiments on inert films of biologics model solutions, lysozyme (Lys) and ribonuclease (RNAse), allowed corroborating the printing system capacity to deliver proportional amounts to the printed areas and concentrations of inks (Montenegro Nicolini M, Miranda V, Campano FA, Toro E, Morales JO The Use of Inkjet Printing for Dosing Biologics on Drug delivery Systems In: 2015 AAPS Annual Meeting and Exposition Orlando, Florida, USA, 2015).
Additionally, experimental studies showed that the printing process can reduce the integrity of the drug (measured as remaining activity) and proportional to the amount printed. Similarly, the incorporation of an absorption enhancer has a negative effect on the integrity of the biologic, decreasing its activity (Montenegro-Nicolini M, Miranda V, Toro E, Morales JO. The Use of Inkjet Printing for Dosing biomacromolecular Actives in Drug Delivery Systems in: 42nd Annual Meeting and Exposition of the Controlled Release Society Edinburgh, Scotland, 2015), see Figure 3.
Experimental results of nanoemulsions and nanocapsules, were designed to find formulations useful as printing inks regarding sizes, uniformity, Lys encapsulation, and post-printing activity. The design was conducted as ternary diagram design of how the composition results in different conditions for nanoemulsions (Table 1 and Figure 4).

**Table 1. Of the total formulations of the ternary diagram, here are shown those formulations that resulted in satisfactory nanoemulsions in terms of size, polydispersity and zeta potential.**

| %Miglyol | %Lecithin | %Water | Size/nm | PDI | Zeta potential |
|---|---|---|---|---|---|
| 2,31 | 0,58 | 97,1 | 93,4 ± 1,0 | 0,15 ± 0,03 | -52,0 ± 2,0 |
| 10 | 10 | 80 | 280,0 ± 2,3 | 0,38 ± 0,01 | -66,7 ± 1,3 |
| 20 | 20 | 60 | 330,3 ± 6,7 | 0,39 ± 0,02 | -72,6 ± 0,8 |
| 5 | 5 | 90 | 205,3 ± 4,0 | 0,27 ± 0,02 | -79,1 ± 0,5 |
| 2 | 2 | 96 | 172,9 ± 2,9 | 0,21 ± 0,02 | -53,7 ± 1,8 |
| 1 | 1 | 98 | 244,2 ± 16 | 0,32 ± 0,02 | -54,2 ± 0,6 |
| 1 | 0,1 | 98,9 | 156,7 ± 1,7 | 0,09 ± 0,02 | -37,0 ± 0,4 |

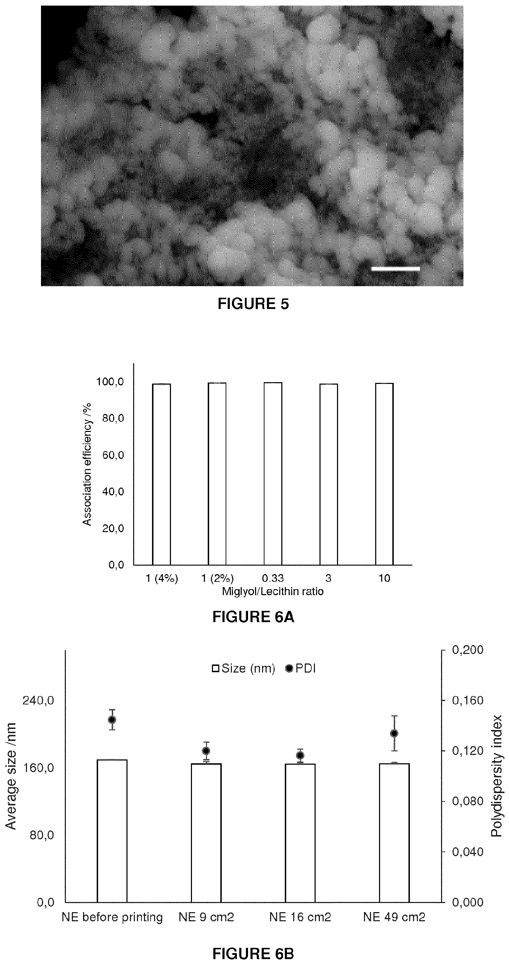

Table 1 shows that increasing the oil phase (miglyol) and surfactant (lecithin) the particle size is increased, but an increase is observed in the polydispersity (PDI). One can also conclude that when approaching the proportions of the reference formulation (miglyol 2.31% and 0.58% lecithin) the size increases with a decrease of PDI.
However, and for purposes of inkjet printing, the properties of these nanoemulsions allow them to be printed (e.g., Table 1). Printing nanoemulsions loaded with lysozyme is possible and the physicochemical characteristics of the particles are held after completion of the printing process. Figure 6a shows that the printing process does not affect the load (association efficiency and loading) of a biologic model (lysozyme) in nanoemulsions formulated with different miglyol/lecithin relationships. Furthermore, it can be seen in Figure 6b that the physicochemical characteristics of lysozyme loaded nanoemulsions are not modified after the printing process.

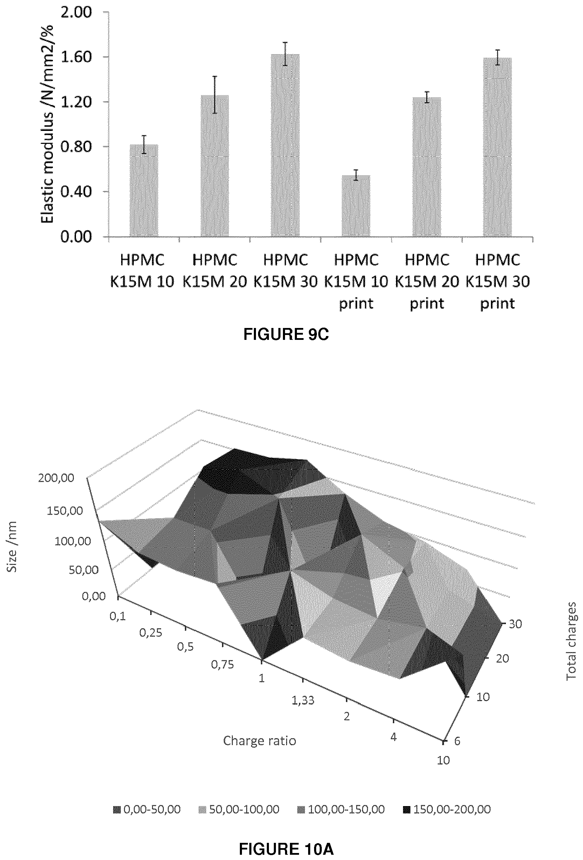

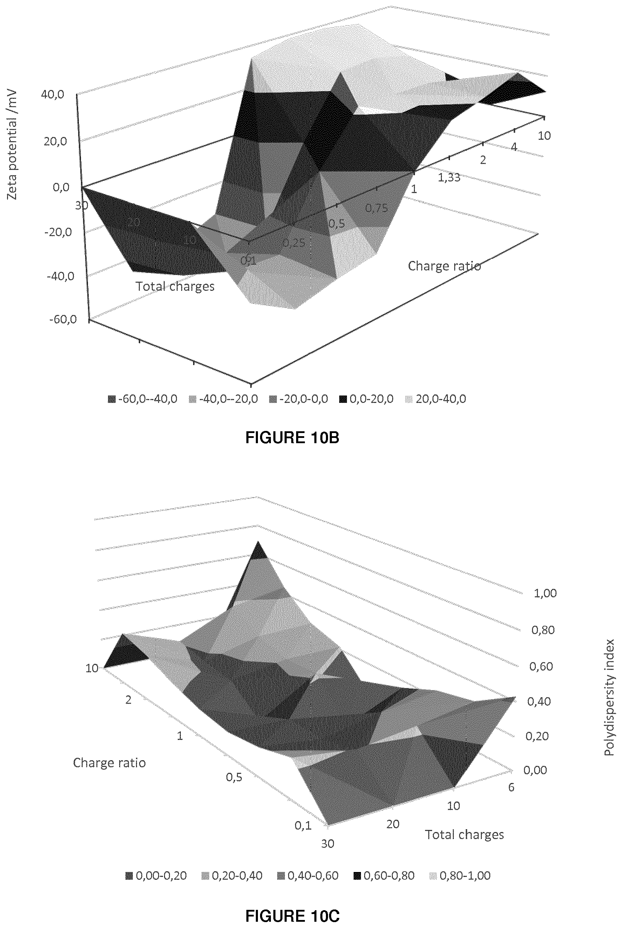

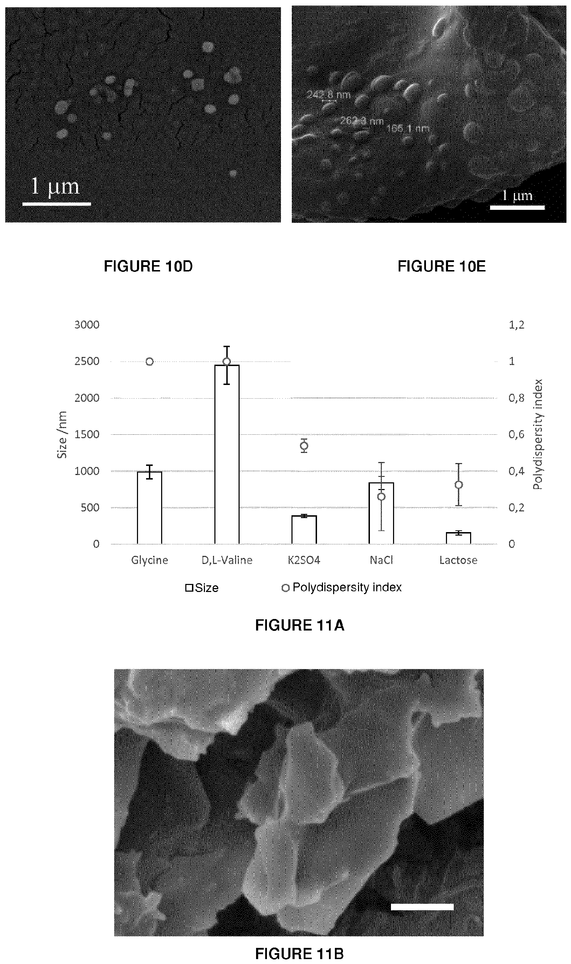

Due to their potential, polymeric nanoparticles were studied to deliver a peptide with cardiovascular effects (angiotensin 1-9) (Sepulveda-Rivas S, Gomez MT, Morales JO Toxicity Evaluations of Polymeric Nanoparticles: Cell Viability versus Physicochemical Properties of Nanoparticles In: 2015 AAPS Annual Meeting and Exposition Orlando, Florida, USA, 2015; Morales JO, Sepulveda-Rivas S, Oyarzun-Ampuero F, Lavandero S, Kogan MJ Novel nanostructured polymeric carriers to enable drug delivery for cardiovascular diseases. Curr Pharm Des 2015; 21 (29): 4276-84), nanoparticles for delivery to the central nervous system (Catalan-Figueroa J, Jara MO, Gajardo-Lopez U, Morales JO Polymeric Nanocarriers for Drug delivery: Improving physicochemical parameters. in: NanoDDS Meeting 2015 Seattle, Washington, USA; 2015; Catalan-Figueroa J, Palma-Florez S, Alvarez G, H Fritz, MO Jara, Morales JO Nanomedicine and nanotoxicology. Central Nervous System considerations. Nanomedicine. 2015; Accepted; Gajardo-Lopez U, Jara MO, JO Morales. Nanoprecipitation of Eudragit RS and RL to Fabricate Nanostructured delivery Systems for Curcumin. In: Annual AAPS 2015 Meeting and Exposition. Orlando, Florida, USA; 2015; Jara MO, Landin M, Morales JO. A Systematic Analysis of the Manufacture of Polymeric Nanoparticles by Nanoprecipitation (Solvent Diffusion) by Artificial Neural Networks. In: Annual AAPS 2015 Meeting and Exposition. Orlando, Florida, USA; 2015), and nanosuspensions for use as printing inks (Sepulveda-Rivas S, Gomez MT, Morales JO Toxicity Evaluations of Polymeric Nanoparticles: Cell Viability versus Physicochemical Properties of Nanoparticles In: 2015 AAPS Annual Meeting and Exposition Orlando, Florida. , USA, 2015; Fritz H, Morales JO The Use of Eudragit E/Alginate Polymeric Nanoparticles as a Novel Drug delivery System for Biomacromolecules. a Case Study of Lysozyme in: 2015 AAPS Annual Meeting and Exposition Orlando, Florida, USA; 2015).

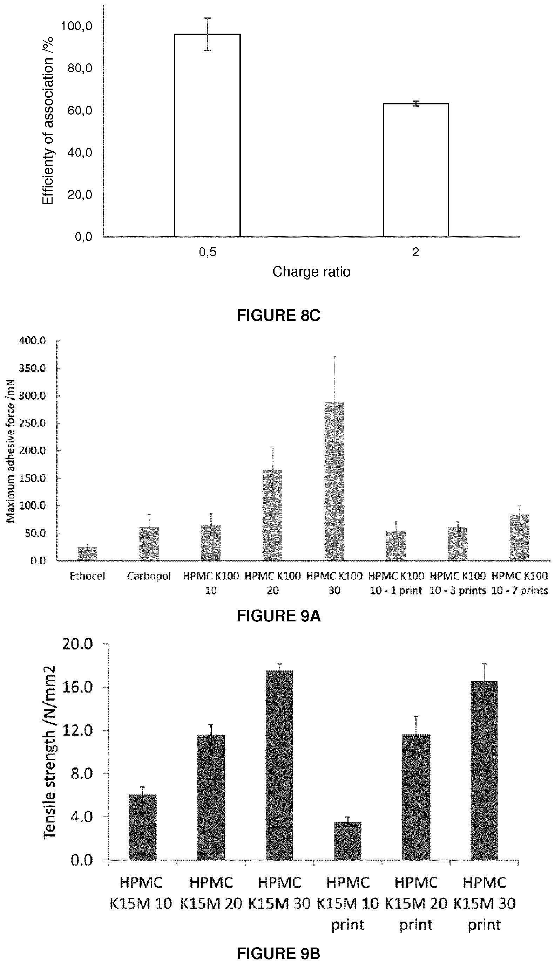

Figure 8a shows that the printing process does not alter the physicochemical characteristics of polymeric nanoparticles obtained by coacervation of Eudragit E and alginate at different charge ratios (CR) and different total charges (TC) in the number of positive and negative charges which provide each polymer forming the nanoparticle. Figure 8b shows that a controlled and reduced release can always be maintained in the first 72 hours regardless of CR and TC conditions and after subjecting the particles to printing process. This profile ensures that such nanocarriers can carry the drug protected from environmental conditions (and the printing process) to and towards the therapeutic target where it can be released. Figure 8c shows the typical appearance of these spherical polymeric nanoparticles.
Experimental studies with various types of hydroxypropylmethyl cellulose (HPMC K3, K100, and K15M) showed that after the printing process, the mechanical and morphological properties of the polymer films do not vary (Alvarez R, Bull E, Beltran F, Fernandez M, Morales JO Study of polymeric films as substrates for inkjet printing of drug delivery systems In: AAPS National Biotechnology Conference 2014 San Diego, California, USA, 2014) (Figure 9). The mucoadhesive properties are diminished, but do not prevent the use of the film as a dosage form.
Other cellulose derivatives can also be used such as hydroxypropylcellulose, hydroxyethylcellulose, carboxymethylcellulose.
Experimental studies of polycation and polyanion (n⁻/n⁺) and the sum of total charges available to the reaction (in Table 2, n⁻ + n⁺ = 6 µM) to determine their influence on the characteristics of the nanoparticles show a trend to lower average sizes as the charge ratio increases, relatively independent of the total charge. The smaller sizes become apparent from a charge ratio of 0.5 (Figure 9a), which coincides with the observed in zeta potential, where the ratio of 0.5 is where the inversion of negative (predominance of polyanion) to positive charges (predominance of polycation) (Figure 9b) occurs. However, the results of the polydispersity index indicate that the region between charge ratio 0.25 to 0.75 combines smaller sizes, stronger zeta potential, and low polydispersity indices (Figure 10a-c). Inspection of particle morphology by scanning electron microscopy (SEM) allowed visualization of the spherical aspect of these polymeric nanoparticles and their correlation with the size determined by dynamic light scattering (DLS) (Figure 10d and Figure 10e).

**Table 2. Effect of charge ratio between Eudragit E PO (polycation) and alginate (polyanion) on the properties of size, polydispersity, and zeta potential of polymeric nanoparticles. This table corresponds to the total sum of charges (n⁻ + n⁺) equal to 6 µM in experiments exploring the range comprised of 2, 4.4, 6, 10 and 20 µM. Results are expressed as mean (standard deviation).**

| Charge ratio (n⁺/n⁻) | Size (nm) | Polydispersity index | Zeta potential (mV) |
|---|---|---|---|
| 0,1 | 123,8 (4,9) | 0,213 (0,015) | -33,2 (2,4) |
| 0,25 | 137,6 (2,6) | 0,112 (0,001) | -33,2 (0,6) |
| 0,5 | 119,5 (2,0) | 0,149 (0,009) | 40,0 (1,2) |
| 0,75 | 76,2 (3,3) | 0,291 (0,034) | 37,8 (2,6) |
| 1 | 56,8 (1,0) | 0,283 (0,023) | 36,1 (3,0) |
| 1,33 | 59,1 (0,8) | 0,216 (0,007) | 27,5 (36,6) |
| 2 | 57,8 (7,0) | 0,330 (0,075) | 62,6 (32,7) |
| 4 | 66,2 (3,5) | 0,366 (0,012) | 87,6 (39,5) |
| 10 | 92,9 (19,5) | 0,639 (0,046) | 30,9 (14,7) |

The results of experimental studies for the use of nanoparticles incorporated in the conventional manner into polymeric films, as buccal delivery system, were developed with insulin as a model biologic, a potential therapy for treating diabetes. The results demonstrate the present invention relates to the manufacture of protein coated nanoparticles that can be developed by varying the molecule constituting the core coprecipitation.
Table 3 shows results obtained using various concentrations of sorbitan monostearate (SMS, as surfactant) with saccharides such as lactose, mannitol and sorbitol. In terms of size and polydispersity (Pdl), a control in size and dispersion was observed for lactose and mannitol formulations, whereas the use of sorbitol only allows tighter control at low SMS concentrations (Le, A.-D., Berkenfeld, K., Elmaoued, A., Morales, JO, and McConville, JT (2014) Core Forming Antisolvent Coprecipitation of Protein Crystals Loaded, in 2014 AAPS Annual Meeting and Exposition. San Diego, California, USA)

**Table 3. Effect of co-precipitant type (core precursor) on the average size and polydispersity index at different surfactant concentrations (SMS).**

| | Lactose | | Mannitol | | Sorbitol | |
|---|---|---|---|---|---|---|
| Sorbitan mono stearate (SMS) conc. [µM] | Size (nm) | Polidispersity index (PdI) | Size (nm) | Pdl | Size (nm) | PdI |
| 0 | 150,1 | 0,061 | 184,1 | 0,245 | 185,7 | 0,162 |
| 8 | 151,9 | 0,170 | 185,4 | 0,248 | 165,3 | 0,218 |
| 15 | 138,7 | 0,080 | 186,0 | 0,094 | 519,1 | 0,735 |
| 30 | 146,1 | 0,175 | 175,0 | 0,138 | 488,2 | 0,814 |
| 45 | 134,1 | 0,220 | 185,6 | 0,279 | 533,9 | 0,404 |
| 60 | 149,8 | 0,102 | 159,6 | 0,150 | 162,5 | 0,322 |

The results indicate that only some core materials are compatible with the process of coprecipitation by antisolvent (such as the aforementioned saccharides, ie, lactose, mannitol and sorbitol) according to the properties of average size and zeta potential (Le, A. -D., Morales, JO, Berkenfeld, K., and McConville, JT (2014) Co-precipitation Antisolvent Synthesis of D,L-Valine/Lysozyme, in 2014 AAPS Annual Meeting and Exposition. San Diego, California, USA).
In experimental studies of nanoemulsions and nanocapsules as vehicles for lysozyme, systems were obtained by the method of solvent displacement, where the organic phase containing oil (as an oil core of the nanostructure), a surfactant, and water-miscible organic solvents (such as acetone or ethanol), is incorporated into an aqueous solution to form a nanoemulsion (without agitation or high-energy homogenization). Particularly, experimental studies for incorporating lysozyme as biologic model or example in nanoemulsions and nanocapsules as potential buccal delivery system were performed. Table 4 shows the zeta potential inversion from the nanoemulsion (expressing the negatively charged surfactant) to Eudragit EPO nanocapsules (polycation). Better encapsulation efficiency is observed at low lysozyme loads, indicating that the emulsification system has a maximum capacity of incorporation.

**Table 4. Size, polydispersity index, zeta potential and encapsulation efficiency (EE) of nanoemulsions and nanocapsules containing lysozyme formulations. Results expressed as mean (standard deviation).**

| Formulation | Size (nm) | Polydispersity index | Zeta potential (mV) | EE (%) |
|---|---|---|---|---|
| NE + Lys 1% | 169,2 (0,4) | 0,14 (0,01) | -12,8 (0,4) | 81,4 (0,4) |
| NC EPO 0,1% + Lys 1% | 209,8 (0,9) | 0,20 (0,01) | 83,2 (0,5) | 87,0 (2,4) |
| NC EPO 0,05% + Lys 1% | 193,7 (3,3) | 0,14 (0,01) | 84,4 (2,3) | 109,9 (5,0) |
| NE + Lys 10% | 200,5 (1,4) | 0,13 (0,02) | -38,5 (1,2) | 39,5 (1,3) |
| NC EPO 0,1% + Lys 10% | 226,3 (1,6) | 0,20 (0,01) | 76,1 (1,7) | 18,0 (0,8) |
| NC EPO 0,05% + Lys 10% | 187,9 (0,7) | 0,27 (0,01) | 83,4 (0,3) | 17,8 (0,1) |

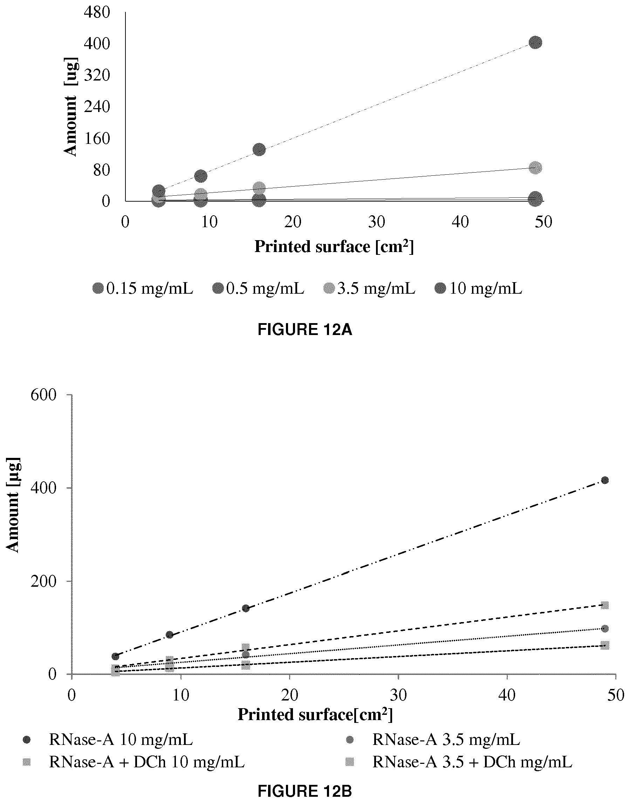

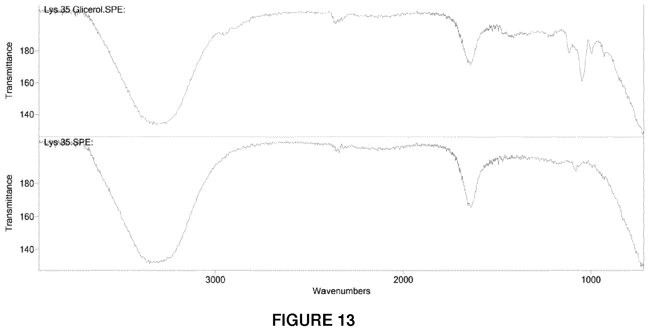

Studies of printing inks containing lysozyme or ribonuclease (RNAse) as biologics models or examples demonstrated that the printing process allows obtaining controlled doses and increasing linearly with respect to the printed surface. Additionally, the incorporation of an absorption enhancer such as deoxycholate, maintains the same linear trends in the printing process with lysozyme but with a decrease in the amount printed under the same conditions of surface and lysozyme concentration (Figure 12). In general, the absorption enhancer may be selected from bile salts and their derivatives, including taurocholate, glycodeoxycholate, glycocholate, and taurodeoxycholate. Since inks require the addition of glycerol as a viscosity agent, the evaluation of lysozyme inks was performed with or without glycerol by infrared spectroscopy Fourier transform (FTIR). An inspection of the major bands of the spectra shows that lysozyme does not change during the process (Figure 13).
Experimental studies for the evaluation of the printing process on biologics nanoparticles as vehicles, were performed manufacturing polymeric nanoparticles and incorporated into the printing cartridge as a nanosuspension. The particle properties were measured before and after the printing process. Except an increase in the polydispersity index, the particles maintain their properties during the printing process and define the methodology for incorporating drug loaded nanoparticles on polymer films (Table 5).

**Table 5. Effect of the printing process on polymeric nanoparticles (NPs) in a model ink. Results expressed as means (standard deviation).**

| Test condition | Size (nm) | Zeta potential (mV) | Polydispersity index |
|---|---|---|---|
| NPs before printing | 214,3 (1,3) | -34,1 (0,3)* | 0,17 (0,02)** |
| NPs after printing | 226,0 (17,8) | -26,0 (3,8)* | 0,43 (0,11)** |

| | | | |
|---|---|---|---|
| *,**: differences observed are statistically significant (p<0,05) | | | |

Experimental studies for the development of nanocarriers constituting printing inks containing biologics as models or examples of the present invention focused on the fabrication and characterization of nanoparticles by various techniques. The techniques employed were selected from the group consisting of: manufacturing polymeric nanoparticles (also referred to as nanocomplexes), protein coated nanoparticles (protein-stabilized nanoparticles), nanoemulsions, and nanocapsules.
Subsequent experiments with a synthetic polycation, a polymethacrylate derivative with ionizable tertiary amino groups in its monomeric units (Eudragit® E PO), resulted in nanoparticles with excellent size, polydispersity index and zeta potential.

### Examples of nanosuspension printing as inks

Nanosuspensions of polymeric nanoparticles loaded with lysozyme as printing inks were developed as follows: controlled amounts of Eudragit® E solutions (EPO) 2.5 mg/mL (a cationic derivative of polymethacrylate) were mixed, under constant magnetic stirring (300 rpm) at room temperature, with defined amounts of alginate solutions 2.5 mg/mL (anionic polymer) according to the following Table:

| Positive charges (n+) | NEgative charges (n-) | Charge ratio | Total charge sum (n⁺ + n⁻) | Conc EPO | Conc Alg | Vol EPO | Vol Alg | Size (SD) | |
|---|---|---|---|---|---|---|---|---|---|
| µmol charges | µmol charges | n+/n- | µmol charges | µg/µL | µg/µL | µL | µL | nm | |
| 27,3 | 2,7 | 10 | 30 | 2,5 | 2,5 | 3.032,7 | 216,1 | 140,7 | 2,2 |
| 24 | 6 | 4 | 30 | 2,5 | 2,5 | 2.668,8 | 475,5 | 132,4 | 3,7 |
| 20 | 10 | 2 | 30 | 2,5 | 2,5 | 2.224,0 | 792,4 | 143,2 | 6,1 |
| 10 | 20 | 0,5 | 30 | 2,5 | 2,5 | 1.112,0 | 1.584,9 | 236,4 | 4,0 |
| 6 | 24 | 0,25 | 30 | 2,5 | 2,5 | 667,2 | 1.901,9 | 212,7 | 5,0 |
| 2,7 | 27,3 | 0,1 | 30 | 2,5 | 2,5 | 303,3 | 2.161,2 | 191,2 | 13,2 |

Based on the mass of polymer reaction, 10% w/w lysozyme was included in each formulation from a stock solution of 1 mg/mL of lysozyme. 1.5 mL of each of the resulting nanosuspensions (different formulations) were centrifuged at 13000 rpm for 30 minutes on 50 µL of glycerol (for redispersing). After removing the supernatant, the pellet was resuspended with an aqueous solution with 30% v/v of glycerol as ink viscosity agent. These nanosuspensions are the printing inks. Additionally, these nanosuspensions can be added with an absorption enhancer such as deoxycholate.
These inks were loaded into cartridges compatible with HP Deskjet 1000 printer and installed to run the printing routines. Using a commercial software tool appropriate for the task, surfaces 3 x 3 cm² were printed on an inert substrate to assess the effect of printing on the polymeric nanoparticles. These printed amounts were collected by refluxing with 1 mL of milliQ water. The physicochemical properties in terms of size, polydispersity index and zeta potential of these nanoparticles were determined after recovering after the printing process and results are described in Figure 2A-2C. Similarly, insulin nanosuspensions formulations were prepared by combining controlled amounts of solutions Eudragit E (EPO) 2.5 mg/mL, under constant magnetic stirring (300 rpm) at room temperature, with defined amounts of alginate solutions 2.5 mg/mL according to the following Table:

| Positive charges (n+) | NEgative charges (n-) | Charge ratio | Total charge sum (n⁺ + n⁻) | Conc EPO | Conc Alg | Vol EPO | Vol Alg | Size (SD) | |
|---|---|---|---|---|---|---|---|---|---|
| µmol charges | µmol charges | n+/n- | µmol charges | µg/µL | µg/µL | µL | µL | nm | |
| 20 | 10 | 2 | 30 | 2.5 | 2.5 | 2224.0 | 792.4 | 191.9 | 4.5 |
| 10 | 20 | 0.5 | 30 | 2.5 | 2.5 | 1112.0 | 1584.9 | 182.4 | 1.7 |

Based on the mass of polymer reaction, 10% w / w insulin was included in each formulation from a stock solution of 1 mg/mL. 1, 5 mL of each of the resulting nanosuspensions (different formulations) were centrifuged at 13000 rpm for 30 minutes on 50 µL of glycerin (to redisperse). After removing the supernatant, the pellet was resuspended with an aqueous solution with 30% v/v glycerol as ink viscosity agent. These nanosuspensions are printing inks. Additionally, these nanosuspensions can be added with an absorption enhancer such as deoxycholate.
These inks were loaded into cartridges compatible with HP Deskjet 1000 printer and installed to run the printing routines. Using a commercial software tool appropriate for the task, surfaces 3 x 3 cm² were printed on an inert substrate to evaluate the effect of printing on the polymeric nanoparticles. These printed surfaces were collected by refluxing with 1.0 mL of milliQ water. The physicochemical properties in terms of size, polydispersity index and zeta potential of these nanoparticles recovered after the printing process were determined and results are described in Figure 8A-B.

### Printing example of biologies

To evaluate the effect of printing on biologics, RNAse and lysozyme solutions were prepared and subjected to the printing process. Aqueous solutions (milliQ water) of lysozyme were prepared at 0.15, 0.5, 3.5, and 10 mg/mL. Alternatively, solutions with the same concentrations of lysozyme were spiked with 25% w/w deoxycholate based on the mass of lysozyme as an conventional absorption enhancer. To constitute inks, these aqueous solutions were mixed in a 7:3 v/v of solution/gllcerol as ink viscosity agent.
Similar to as indicated above, these solutions were printed on an HP Deskjet 1000 at 4, 9, 16, and 49 cm². These printed surfaces were collected by refluxing with 1 mL of milliQ water and the amount of printed lysozyme (Figure 3A and 3B) and its remaining activity (similar to Figure 4C related lysozyme) were quantified.
For studies with RNAse, aqueous solutions (milliQ water) of RNAse were prepared at 0.15, 0.5, 3.5, and 10 mg/mL. Alternatively, solutions with the same concentrations of RNAse were added with deoxycholate 25% w/w as an conventional absorption enhancer based on the mass of RNAse. To constitute the inks, these aqueous solutions were mixed in a 7:3 v/v of solution/glycerol as ink viscosity agent.
Similar to as indicated above, these solutions were printed on an HP Deskjet 1000 at 4, 9, 16, and 49 cm². These printed surfaces were collected by refluxing with 1 mL of milliQ water and the amount printed RNAse (Figure 12A and 12B) and its remaining activity (Figure 4A and 4B) were quantified.

### Printing example of polymeric films

To evaluate the effect of model printing routines on polymer films for buccal drug delivery, hydroxypropylmethyl cellulose (HPMC) films of different grades were manufactured.
Aqueous solutions of HPMC K3, K100, and K15M 10% w/v were prepared and over solid polymer base, films were incorporated with 10% w/w glycerol as plasticizer in the casting solution. Constant magnetic stirring (500 rpm) at room temperature was used for the dissolution until a translucent solution. After complete dissolution of the polymer, these solutions were stored at 4 °C for 24 to remove air bubbles from the solution. Subsequently 10, 20, or 30 g of these solutions were casted in polytetrafluoroethylene molds (PTFE or Teflon) and left to dry in an air current until constant weight for at least 24 hours. Subsequent to drying, the polymer films were demolded and used in the printing process.

As model inks, aqueous solutions of glycerol 30% v/v were used and the effect that the process of printing on films in terms of mechanical properties such as elongation at break, tensile strength, and elastic modulus (Figure 9B and 9C) was evaluated. Due to the potential of these films as dosage forms for buccal administration, their pre and post printing mucoadhesive properties were evaluated and compared to Carbopol and ethylcellulose, other polymers with different mucoadhesive properties and conventionally used (Figure 9A).

## Claims

1. Dosage form for oral administration of highly controlled dose, with a controlled and stable release a biomacromolecule, comprising:
a. a polymer film as printing substrate consisting of at least one pharmaceutically acceptable excipient and
b. an ink printed injection on the polymeric film comprising nanoparticles or nanoparticle suspensions comprising said biomacromolecule and at least one pharmaceutically acceptable excipient, wherein said biomacromolecule is selected from a lymphokine a hormone, hematopoietic factors, growth factors, antibodies, enzymes, inhibitors and vaccines;
wherein said lymphokine is selected from aldesleukin cytokine, the antineoplastic protein denileukin difititox, the recombinant interleukin Oprelvekin, interferon α1, interferon α2a, interferon-α2b, interferon β1a, interferon β1b, interferon γ1b, and human-α1a tumoral necrosis factor (TNFα-1a) tasonermin;
wherein said hormone is selected from human insulin, insulin lispro, insulin aspart, insulin glulisine, insulin glargine, insulin detemir, glucagon, somatropin, somatrem, follitropin-α, foliitropin-β, choriogonadotropin-α, lutropin-α, calcitonin, teritapide, Preotact, thyrotropin-α, nesiritide;
wherein said hematopoietic factors are selected filgrastim, lenogastrim, sargramostim, molgramostim, epoetin-α, epoetin-β, γ-epoetin, darbepoetin-α;
wherein said growth factors are selected from mecasermin, rinfabate mecarsemina, nepiderine, becaplermin, palifermin, dibotermin-α, epotermina-α,
wherein said antibodies are selected from Fab fragments including arcitumomab, digoxin Fab, abciximab, certolizumab; muramonab-CD3 antibodies including murine, ProstaScint, ibritumomab tiuxetan, toistumomab; Chimeric antibodies including rituximab, infliximab, basiliximab, cetuximab, brentuximab vedotin; daclizuman including humanized antibodies, trastuzumab, palivizumab, gemtuzumab ozogamicin, alemtuzumab, efaüzumab, omalizumab, bevacizumab, natalizumab, ranbizumab, eculizumab, tociiizumb; human antibodies incuyendo adalimumab, panitumimab, golimumab, Canakinumab, ustekinumab, ofatumumab, denosumab, beiimumab, ipiiimumab;
wherein said enzymes are selected from imiglucerase, agalsidase-β, Alglucosidase-α, laronidase, aldusufasa, galsuifasa factor VIIa, factor VIII, factor IX, drotrecogin-α, alteplase, reteplase, teneceplasa, dornase-α, rasburicase;
where such inhibitors are selected desirudin, lepirudin, antithrombin III, ecallantide, anakinra;
where such vaccines are selected from human hepatitis vaccine, human papillomavirus vaccine, and
wherein said biomacromolecule is lysozyme, ribonuclease, angiotensin 1 -9 or insulin.

2. The dosage form of claim 1, wherein said polymer film comprises film forming polymers including polyvinyl pyrrolidone, polyvinyl alcohol, chitosan, alginate, agar, carrageenan, guar gum, xanthan gum, polycarbophil, polyacrylic acid derivatives, and derivatives polymethacrylic acid.

3. The dosage form of claim, wherein said polymeric film is a polymethacrylic acid derivative.

4. The dosage form of claim 3, wherein said derivative of polymethacrylic acid derivative is a cationic polymethacrylate.

5. The dosage form of claim 4, wherein said derivative is cationic Edagrlt polymethacrylate E®

6. The dosage form of claim 2, wherein said film further comprises polymer cellulose derivatives such as pharmaceutically acceptable excipient.

7. The dosage form of claim 1, wherein said film further comprises a polymer plasticizer.

8. The dosage form of claim 1, wherein said ink further comprises an absorption enhancing agent.

9. The dosage form of claim 8, wherein said absorption enhancing agent is a pool salt or derivative thereof.

10. The dosage form of claim 9, wherein said absorption enhancing agent is deoxycholate, turocholate, glicodeoxlcholate, glycocholate and taurodeoxycholate.

11. The dosage form of claim 10, wherein said absorption enhancing agent is deoxycholate.

12. The dosage form of claim 1, wherein the ink further comprises a plasticizer.

13. The dosage form of claim 12, wherein said viscosifying agent is selected from gllcerine, glycerol or propilenglycol.

14. The dosage form of claim 1, wherein said blomacromolecule is lysozime, rlbonuclease, anglotensine 1-9 or insulin.

15. The dosage form of claim 1, wherein said biomacromolecule is lysozime.

16. The dosage form of claim 1, wherein said ribonuclease is blomacromolecule.

17. The dosage form of claim 1, wherein said blomacromolecule is angiotensin 1 -9.

18. The dosage form of claim 1, wherein said blomacromolecule is insulin.

19. The dosage form of claim 1, wherein said nanosuspension comprises a nanoemulsion comprising miglyol, lecithin, water or a mixture thereof as pharmaceutically acceptable excipients.

20. The dosage form of claim 7, wherein said plastlficizer is present in a concentration in the range of 10 to 30% w / w solid base.

21. The dosage form of claim 1, wherein said ink comprises nanoparticles having a size in the range of 50 to 500 nm.

22. The dosage form of claim 21, wherein said nanoparticles have a size in the range of 90 to 350 nm.

23. The dosage form of claim 1, wherein said ink comprises nanoparticles selected from the group consisting of: polymer nanoparticles, nanoemulsions and nanocapsules, nanoparticles and coated.

24. The dosage form of claim 23, wherein said coated nanoparticles have a size in the range of 50 to 150 nm.

25. The dosage form of claim 24, wherein said coated nanoparticles are nanoparticles coated with proteins, which are obtained by precipitation of biomacromolecules.

26. The dosage form of claim 23, wherein said coated nanoparticles further comprises saccharides as a co-agent precipitation.

27. The dosage form of claim 26, wherein said saccharide is selected from the group consisting of lactose, manltol and sorbitol.

28. The dosage form of claim 1, wherein said ink comprises nanoemulsions having a size in the range of 150 to 200 nm.

29. The dosage form of claim 28, wherein said nanoemulslons comprise 1 to 10% lysozime as blomacromolecule.

30. The dosage form of claim 1, wherein said ink comprises nanocapsules having a size in the range of 150 to 250 nm.

31. The dosage form of claim 30, wherein said nanoemulslones comprise 1 to 10% lysozime as blomacromolecule and 0.05% to 0.1% of at least one surfactant.

32. The dosage form of claim 31, wherein said surfactant is lecithin.

33. Method for the manufacture of the dosage form of claim 1, comprising the steps of:
a) preparing nanoparticles or nanoparticle suspensions biomacromolecules,
b) preparing a suspension of printing ink with said nanoparticles,
c) printing polymer films with said suspension of nanoparticles biomacromolecules.

34. The method of claim 33, comprising preparing nanoparticles selected from the group consisting of: polymer nanoparticles, and nanocapsules, nanoemulsions and coated nanoparticles.

35. The method of claim 34, wherein said protein coated nanoparticles are prepared by co-precipltation by antlsolvent.

36. The method of claim 34, wherein said polymeric nanoparticles are prepared by coacervation.

37. The method of claim 37, wherein said polymeric nanoparticles are prepared by complex coacervation where polycatlon including a polymer with cationic monomer units is made to interact electrostatically with a polyanion including a polymer with anionic monomeric units in a charge ratio of 1 to 1.5 polyanion to polycation.

38. The method of claim 37, wherein said polycation is a synthetic polycation, polymethacrylate derivative with ionizable tertiary amino groups in its monomeric units, Eudragit® EPO.

39. The method of claim 37, wherein said nanoparticles are prepared by nanoprecipitation.

40. The method of claim 37, wherein said nanoemulsions and nanocapsules as said biomacromolecule vehicles were prepared by the solvent displacement method, where an organic phase containing oil as the oily core of the nanostructure, a surfactant including lecithin, chloride cetylpyridinium, hexadecyltrimethylammonium bromide, and water-miscible organic solvents including acetone or ethanol, is incorporated into an aqueous solution to form a nanoemulsion without stirring or homogenisation energy.
